# EUROPÄISCHE PATENTSCHRIFT

(11) **EP 2 882 887 B1**
(45) Veröffentlichungstag und Bekanntmachung des Hinweises auf die Patenterteilung: **31.10.2018**
(21) Anmeldenummer: 13759970.0
(22) Anmeldetag: 07.08.2013
(51) Int. Cl.: C23G 1/00, G01N 1/34, G01N 33/20, G01N 21/88

(54) **OBERFLÄCHENBEHANDLUNGSVERFAHREN FÜR BAUTEILE AUS ALUMINIUM MIT EINEM NACHWEIS EINER UNZULÄSSIGEN ÜBERHITZUNG**
SURFACE TREATMENT PROCESS FOR COMPONENTS COMPOSED OF ALUMINIUM HAVING DETECTION OF IMPERMISSIBLE OVERHEATING
PROCÉDÉ DE TRAITEMENT DE SURFACE POUR ÉLÉMENTS EN ALUMINIUM, COMPORTANT UNE DÉTECTION D'UNE SURCHAUFFE INADMISSIBLE

(30) Priorität: 08.08.2012 DE 102012015579
(43) Veröffentlichungstag der Anmeldung: 17.06.2015
(73) Patentinhaber: Premium AEROTEC GmbH, 86179 Augsburg (DE)
(72) Erfinder: JURICIC, Claudia, 81929 München (DE); MARKGRAF, Melanie, 86199 Augsburg (DE)
(74) Vertreter: LKGLOBAL Lorenz & Kopf PartG mbB Patentanwälte
(86) Internationale Anmeldenummer: PCT/DE2013/000442
(87) Internationale Veröffentlichungsnummer: WO 2014/023283

(56) Entgegenhaltungen:
- EP-A1- 0 898 163
- WO-A1-2010/006895
- DE-A1- 3 340 706
- US-A- 4 383 042
- US-B1- 7 126 123
- CERACKI P ET AL: "ONLINE-FEHLERERKENNUNG BEI WARMBAND DURCH AUTOMATISCHE OBERFLAECHENINSPEKTION", STAHL UND EISEN, VERLAG STAHLEISEN, DUSSELDORF, DE, Bd. 119, Nr. 4, 15. April 1999 (1999-04-15), Seiten 77-81,164, XP000830261, ISSN: 0340-4803

## Beschreibung

Die Erfindung betrifft ein Oberflächenbehandlungsverfahren gemäß Anspruch 1, insbesondere ein Oberflächenschutzverfahren bzw. ein Oberflächenbeschichtungsverfahren mit einer Überprüfung einer unzulässigen lokalen Überhitzung für Bauteile bzw. von Bauteilen aus Aluminium oder Aluminiumlegierungen, das einen Vorprozess mit einem alkalischen Reinigen und/oder alkalischen Beizen als Teilprozessschritt enthält. Sie betrifft außerdem ein Verfahren zur Prüfung derartiger Bauteile auf Weichfleckigkeit in einem Oberflächenbehandlungsverfahren.

Der Begriff "Weichfleckigkeit", im Englischen "weak spot", "hot spot" oder "partial material affection" (PMA) genannt, bezeichnet eine unzulässige lokale Überhitzung, die beim Zerspanen, insbesondere bei hochfesten Aluminiumlegierungen auftreten kann. Die Überhitzung kann bei ungünstig gewählten Zerspanparametern, Werkzeugversagen oder im Falle verminderter Wärmeableitung entstehen. Sie führt zu einem lokalen Abfall der Härte des Bauteils, der mechanischen Festigkeiten und zu einer lokalen Änderung der elektrischen und thermischen Leitfähigkeit. "Weiche Flecken" lassen sich entweder durch Messungen der Härte, der elektrischen Leitfähigkeit oder durch thermografische Untersuchungen detektieren. Aufgrund des dafür erforderlichen erheblichen Aufwands wird jedoch in der Praxis häufig lediglich eine Sichtprüfung vorgenommen. Denn "weiche Flecken" lassen sich beispielsweise nach dem Chromsäureanodisieren "CAA" als deutliche dunkle Flecken ausmachen. Die dunklen Flecken werden durch die Bildung einer dickeren Anodisierungsschicht im überhitzten Oberflächenbereich des Bauteils hervorgerufen.

Eine vergleichbare Sichtprüfung auf Weichfleckigkeit nach dem abgeschlossenen Anodisierungsvorgang ist jedoch zum Beispiel mit dem umweltfreundlicheren Oberflächenschutzverfahren nach dem Prinzip des Weinsäure-Schwefelsäure-Anodisierens (TSA) nicht mehr möglich. Denn das TSA erzeugt im Gegensatz zum CAA eine transparente Anodisierungsschicht, bei der die Bildung einer dickeren Schicht jedenfalls mit dem bloßen Auge nicht zu erkennen ist. Daher steht im Moment keine für eine Serienprüfung geeignete Prüfmethode für zerspante Bauteile auf Weichfleckigkeit zur Verfügung.

US 4 383 042 A beschreibt ein Oberflächenbehandlungsverfahren für Bauteile aus Aluminium oder Aluminiumlegierungen mit einer visuellen Überprüfung der "Weichflächigkeit", umfassend einen Vorprozess, welches ein alkalisches Reinigen enthält. Aufgabe der Erfindung ist es daher, bei so gut wie allen Oberflächenbehandlungsverfahren für Aluminium und Aluminiumlegierungen, die ein alkalisches Reinigen und/oder alkalisches Beizen umfassen, eine Sichtprüfung auf Weichfleckigkeit vornehmen zu können.

Diese Aufgabe wird durch Anspruch 1 gelöst, wobei bei einem Oberflächenbehandlungsverfahren für Bauteile aus Aluminium oder Aluminiumlegierungen, das die Bauteile in einem Vorprozess den Behandlungsschritten alkalisches Reinigen und/oder alkalisches Beizen unterwirft, nach einem auf das alkalische Reinigen oder auf das alkalische Beizen folgenden Spülen ein separater Schritt der Sichtprüfung auf Weichfleckigkeit durchgeführt wird. Sollte sich bei der Sichtprüfung nach dem auf das alkalische Reinigen folgenden Spülen keine Weichfleckigkeit zeigen, kann auch nach dem auf das alkalische Beizen folgenden Spülen eine weitere Sichtprüfung auf Weichfleckigkeit vorgenommen werden. Der Schritt der Sichtprüfung kann also auch nach dem auf das alkalische Reinigen und nach dem auf das alkalische Beizen folgenden Spülen stattfinden.

Unter Oberflächenbehandlungsverfahren sind unter anderem Oberflächenschutz- bzw. -beschichtungsverfahren zu verstehen wie das Chromsäureanodisieren (CAA = Chromic Acid Anodizing), das Schwefelsäureanodisieren (SAA = Sulfuric Acid Anodizing), das Weinsäure- Schwefelsäureanodisieren (TSA = Tartaric Sulfuric Anodizing), das Phosphor-Schwefelsäureanodisieren (PSA = Phoshoric Sulfuric Anodizing), das Hartanodisieren (HSA = Hard Sulfuric Anodizing) oder das Chemisches Konversionsbeschichten (CCC = Chemical Conversion Coating).

Ein Vorprozess dient insbesondere der Reinigung der Aluminiumbauteile, um anschließend ein erfolgreiches, nämlich insbesondere fehlerfreies Oberflächenschutz- bzw. -beschichtungsverfahren durchführen zu können. Es umfasst regelmäßig die Schritte alkalisches Reinigen, alkalisches Beizen und saures Beizen, wobei zwischen den Schritten jeweils ein einfaches oder mehrmaliges Zwischenspülen (im Folgenden der Einfachheit halber als Spülen bezeichnet) erfolgt. Alkalische Reinigungs- und Beizmittel enthalten chemische Substanzen, die eine alkalische Lösung bilden, beispielsweise NaOH, KOH, Ca(OH)₂. Geeignete Reinigungsmittel werden beispielsweise unter der Bezeichnung P3-Almeco 18 von HENKEL oder unter TURCO 4215 NC von TURCO CHEMIE GmbH vertrieben. Geeignete Beizmittel sind zum Beispiel unter der Bezeichnung ALUMINETCH von HENKEL SURFACE TREATMENTS erhältlich. Weitere Beispiele für alkalische Beizmittel sind der US 4 383 042 zu entnehmen.

Zu den Vorprozessen wird außerdem eine Risseindringprüfung gezählt, die viele Bauteile vor einem Oberflächenschutzverfahren durchlaufen müssen. Da die Bauteile bei dieser Prüfung mit einem Eindringmittel benetzt werden, durchlaufen sie nach Abschluss der Risseindringprüfung erneut einen Vorprozess, um anschließend gereinigt für ein Oberflächenschutzverfahren zur Verfügung zu stehen.

Die Erfindung wendet sich folglich davon ab, eine Sichtprüfung auf Weichfleckigkeit erst nach Abschluss eines Oberflächenbehandlungsverfahrens, beispielsweise erst nach Abschluss eines kompletten Oberflächenschutzverfahrens nach dem Prinzip des Anodisierens oder erst nach einer Risseindringprüfung eines Bauteils vorzunehmen. Sie verfolgt vielmehr das Prinzip, den Vorprozess des betroffenen Oberflächenbehandlungsverfahrens im bzw. nach dem Prozessschritt des alkalischen Reinigens und/oder des alkalischen Beizens zu unterbrechen, um eine Sichtprüfung der Bauteile auf Weichfleckigkeit vorzunehmen. Denn es ist das Verdienst der Erfindung erkannt zu haben, dass sich bereits nach dem alkalischen Reinigen und/oder nach dem alkalischen Beizen die "weichen Flecken" an Stellen zeigen, an denen vorher eine unzulässige Überhitzung stattgefunden hat. Sie zeigen sich nach dem alkalischen Reinigen je nach Blickrichtung und Lichteinfall milchig-weiß bis bräunlich in einer im Übrigen metallischen Fläche. Nach dem alkalischen Beizen werden sie als leicht bräunliche Flecken ggf. in einer kompakten schwarzen Schicht sichtbar, die beim alkalischen Beizen durch das Auszementieren des Kupfers aus der kupferhaltigen Aluminiumlegierung auf der übrigen Bauteiloberfläche entsteht. Das anschließende saure Beizen entfernt die schwarze Schicht samt der bräunlichen Flecken, so dass eine Abweichung in Materialfestigkeit und Gefügestruktur optisch nicht mehr zu erkennen ist. Eine Sichtprüfung wird daher erfindungsgemäß frühestens nach dem alkalischen Reinigen und spätestens vor einem herkömmlichen sauren Beizen bzw. dem vollständigen Entfernen der weitgehend schwarzen Schicht vorgenommen.

Weil die Inspektion auf Weichfleckigkeit nun im Rahmen des Vorprozesses eines Oberflächenbehandlungsverfahrens durchgeführt werden kann, beschränkt sich das erfindungsgemäße Verfahren nicht nur auf ein bestimmtes Oberflächenschutzverfahren, wie vormals beim CAA, bei dem erst nach dem kompletten Prozessdurchlauf die weichen Flecken entdeckt wurden. Es ist vielmehr auf alle Vorprozesse und Oberflächenschutzprozesse anwendbar, bei denen das alkalische Reinigen und/oder alkalische Beizen einen Teilprozess darstellt. Da die Sichtprüfung auf Weichfleckigkeit erfindungsgemäß im Vorprozess stattfindet, muss das Bauteil bei einem Befund, also bei entdeckter Weichfleckigkeit, nicht mehr den kompletten Oberflächenschutzprozess umsonst durchlaufen, sondern kann vorher aussortiert werden. Dadurch können Zeit und Material eingespart werden, wodurch sich das Oberflächenbehandlungsverfahren verbilligt. Viele Bauteile müssen vor einem Oberflächenschutzprozess zudem eine Risseindringprüfung durchlaufen. Die erfindungsgemäße Sichtprüfung auf Weichfleckigkeit kann nun auch vor der aufwendigen Risseindringprüfung durchgeführt werden. Bei einem Befund kann dadurch die Rissprüfung eingespart werden, die anderenfalls überflüssigerweise durchgeführt würde. Auch dadurch lässt sich die Wirtschaftlichkeit des Oberflächenbehandlungsverfahrens steigern.

Die farblichen Veränderungen auf dem Bauteil stehen in einer guten Übereinstimmung mit versuchsweise vorgenommenen Messungen auf elektrische Leitfähigkeit der erfindungsgemäß detektierten "weichen Flecken". So ist innerhalb eines kreisrunden braunen Flecks die elektrische Leitfähigkeit des Werkstoffs reduziert. Bei einer ringförmigen Überhitzung zeigt sich in der Mitte eine höhere elektrische Leitfähigkeit als im Ringbereich. Allerdings beträgt der Abfall der elektrischen Leitfähigkeit im ungünstigsten Fall (lithiumhaltige Aluminiumlegierungen) nur rund 8% und ist nur mit großem Aufwand messtechnisch zu erfassen. Daher ist die Messung der elektrischen Leitfähigkeit bislang nicht als Serienprüfung einsetzbar.

Die Detektion der "weichen Flecken" erfolgt nicht anhand objektiver bzw. objektivierbarer Messergebnisse, sondern im Zuge einer Sichtprüfung. Bei manchen Aluminiumlegierungen ist der farbliche Unterschied zwischen der schwarzen Schicht und den braunen Flecken ausreichend gut zu erkennen. Das Ergebnis der Sichtprüfung kann verbessert werden, indem nach einer vorteilhaften Ausgestaltung der Erfindung vor dem Schritt der Sichtprüfung ein zusätzlicher Schritt der Trocknung des Bauteils nach an sich bekannten Methoden vorgenommen wird. Im trockenen Zustand der Bauteile lassen sich in vielen Fällen die farblichen Unterschiede zwischen den braunen Flecken einerseits und der schwarzen Schicht andererseits deutlicher erkennen. Nach Abschluss der Sichtprüfung kann das getrocknete Bauteil unmittelbar dem Verfahrensschritt des sauren Beizens unterworfen werden. Vorteilhafter Weise wird es jedoch wieder einem vollständigen Vorprozess zugeführt, um spätestens mit dem Verfahrensschritt des sauren Beizens "naß in naß" weiter behandelt zu werden.

Nach einer weiteren vorteilhaften Ausgestaltung der Erfindung kann die Trocknung der Bauteile im zusätzlichen Schritt mittels Druckluft erfolgen. Dadurch lässt sich ein schnelleres Trocknungsergebnis erzielen, das zudem effektiver auf die zu trocknende Bauteiloberfläche gerichtet werden kann. Damit kann der Energieaufwand für die Trocknung verringert werden.

Insbesondere bei einer nur geringfügigen lokalen Überhitzung ergibt ein bloßes Trocknen der Bauteile keinen ausreichend zuverlässig erkennbaren Farbunterschied zwischen den braunen Flecken und der schwarzen Schicht. Daher wird nach der Erfindung die schwarze Schicht teilweise entfernt werden, ohne die braunen Flecken zu beseitigen. Der optisch erkennbare Unterschied zwischen den braunen Flecken einerseits und der schwarzen Schicht andererseits beruht auf einer unterschiedlichen Schichtdicke. Sie ruft durch unterschiedliche Lichtbrechungen einen abweichenden Farbeindruck zwischen der schwarzen Schicht und den braunen Flecken hervor. Letztere entstehen durch eine örtlich höhere Schichtdicke. Durch einen gleichmäßigen Abtrag einer Schicht gleicher Mächtigkeit kann die schwarze Schicht vollständig abgetragen werden, während die weichen Flecken aufgrund der höheren Schichtdicke über ihnen farblich markiert bleiben. Dadurch ergibt sich ein noch deutlicher wahrnehmbarer Farbunterschied zwischen den ungestörten Bereichen eines Bauteils und seinen weichen Flecken. Ein ausreichender, also optisch wahrnehmbarer farblicher Unterschied zwischen der schwarzen Schicht und den braunen Flecken wird schon dadurch erzielt werden, dass die schwarze Schicht nicht vollständig, sondern nur zum Teil abgenommen wird. Je nach verwendeter Technologie für den Abtrag kann ein vorheriges oder nachfolgendes Trocknen entfallen, das Verfahren ggf. also "naß in naß" durchgeführt werden.

Das Entfernen bzw. Abtragen der schwarzen Schicht bzw. eines Teils der schwarzen Schicht in einer gleichmäßigen und einheitlichen Mächtigkeit kann beispielsweise durch mechanischen Verfahren wie Luftdruck-, Wasserstrahl-, Sandstrahl-, Schall- oder Vibrationsverfahren oder durch mechanisches Bürsten erfolgen. All diesen Verfahren ist gemeinsam, dass sie sich flächig anwenden lassen und einen gleichmäßigen flächigen Abtrag der schwarzen Schicht oder eines Teils von ihr ermöglichen. Nach Verfahren gemäß Anspruch 1 wird das Abtragen (eines Teils) der schwarzen Schicht bereits beim vorherigen Spülen im Anschluss an das alkalische Beizen erfolgen. Dazu kann die Spülung bzw. das Wasser in einem Wasserstrahl-Düsenbad mit vielen kleinen Düsen auf das zu spülende Bauteil gerichtet werden. Das Spülen und das Abtragen der schwarzen Schicht kann folglich in einem Schritt des Druckspülens zusammengefasst werden, wodurch sich der erfindungsgemäße Verfahrensablauf vereinfachen, nämlich verkürzen und verbilligen lässt.

Nach einer vorteilhaften Ausgestaltung der Erfindung kann das vollständige oder teilweise Entfernen der schwarzen Schicht zusätzlich oder alternativ durch chemische Mittel erfolgen. Sie lassen sich ebenfalls gleichmäßig verteilt flächig auftragen, so dass eine gleichmäßige Reduktion der Mächtigkeit der schwarzen Schicht oder ihr vollständiger Abtrag erfolgen kann, ohne dass zugleich die verbleibende Schicht auf den "weichen Flecken" entfernt werden würde. Eine Wahl der mechanischen oder des chemischen Verfahrens oder eine Kombination daraus lässt sich anhand von Laborversuchen ermitteln und für den großtechnischen Einsatz auswählen.

Nach einer weiteren vorteilhaften Ausgestaltung der Erfindung kann das Entfernen der schwarzen Schicht mit einer modifizierten sauren Beize erfolgen, wie sie prinzipiell im Vorprozess nach dem alkalischen Beizen ohnehin verwendet wird. Abweichend davon wird jedoch die physikalisch-chemische Einwirkung der modifizierten sauren Beize auf das Bauteil soweit reduziert, dass sie bestenfalls die schwarze Schicht entfernt, nicht jedoch die braunen Flecken. Die Modifikation der erfindungsgemäßen zusätzlichen sauren Beize kann bei im Übrigen unveränderten Verhältnissen durch eine geringere Konzentration, durch eine kürzere Einwirkzeit oder durch eine verringerte Temperatur der sauren Beize herbeigeführt werden. Auch eine Kombination dieser Parameter, beispielsweise eine geringere Konzentration zusammen mit einer kürzeren Einwirkzeit, ggf. alternativ oder zusätzlich mit einer geringeren Temperatur der sauren Beize ist möglich. Alternativ kann einer der Parameter zugunsten der Reduktion eines oder mehrerer anderer Parameter erhöht werden. Beispielsweise kann die Einwirkzeit und/oder die Temperatur einer sauren Beize mit stark herabgesetzter Konzentration deutlich verlängert bzw. erhöht werden. Auch eine kurze Einwirkzeit und/oder hohe Temperatur in Verbindung mit einer erhöhten Konzentration der sauren Beize ist möglich, um die "weichen Flecken" zu erhalten, während die schwarze Schicht entfernt wird. Zusätzlich oder alternativ kann das Beizbad bewegt werden, um die Wirkung eines verringerten Parameters zu steigern.

Da im regulären Schritt des sauren Beizens die schwarze Schicht ohnehin entfernt worden wäre, lässt sich im erfinderischen Verfahren bereits ein Teil dieses Schritts vorwegnehmen, um die Sichtprüfung auf Weichfleckigkeit vornehmen zu können. Der anschließende, reguläre Schritt des sauren Beizens braucht daher nur noch in einem geringeren Umfang vorgenommen zu werden, so dass dadurch Material, Zeit und Energie eingespart werden können.

In den drei vorgenannten Verfahren bildet sich eine schwarze Schicht aus, die zumindest teilweise abgetragen wird, um die "weichen Flecken" deutlicher hervortreten zu lassen. Sie entsteht durch den Schritt des alkalischen Beizens. Nach einer weiteren vorteilhaften Ausgestaltung der Erfindung kann nach einem herkömmlichen "alkalischen Reinigen" und dem nachfolgenden Schritt "Spülen" in einem nachfolgenden Schritt ein Beizen mit einer modifizierten alkalische Beize durchgeführt werden, wobei sich daran die Sichtprüfung und die bekannten Schritte "alkalisches Beizen" und "Spülen" anschließen. Für das alkalische Beizen nach der Sichtprüfung wird unverändert eine herkömmliche alkalische Beize insbesondere mit normaler Konzentration verwendet.

Die Modifikation der erfindungsgemäßen zusätzlichen alkalischen Beize hat den Zweck, eine verringerte bzw. reduzierte physikalisch-chemische Einwirkung der alkalischen Beize auf das Bauteil auszuüben, um nur die "weichen Flecken" anzuzeigen, ohne die schwarze Schicht zu bilden. Die reduzierte Wirkung kann bei im Übrigen unveränderten Verhältnissen durch eine geringere Konzentration, durch eine kürzere Einwirkzeit oder durch eine verringerte Temperatur der alkalischen Beize herbeigeführt werden. Auch eine Kombination dieser Parameter, beispielsweise eine geringere Konzentration zusammen mit einer kürzeren Einwirkzeit, ggf. alternativ oder zusätzlich mit einer geringeren Temperatur der alkalischen Beize ist möglich. Alternativ kann einer der Parameter zugunsten der Reduktion eines oder mehrerer anderer Parameter erhöht werden. Beispielsweise kann die Einwirkzeit und/oder die Temperatur einer alkalischen Beize mit stark herabgesetzter Konzentration deutlich verlängert bzw. erhöht werden. Auch eine kurze Einwirkzeit und/oder hohe Temperatur in Verbindung mit einer erhöhten Konzentration der alkalischen Beize ist möglich, um die "weichen Flecken" zu verdeutlichen, bevor die schwarze Schicht entsteht. Zusätzlich oder alternativ kann das alkalische Beizbad bewegt werden, um die Wirkung eines verringerten Parameters zu steigern.

Eine geeignete Konzentration des modifizierten Beizmittels liegt beispielsweise zwischen 9g bis 18g Beizmittel auf 1 Liter destilliertes Wasser im Gegensatz zu einer Konzentration eines herkömmlichen Beizbads von 38g/l. Seine Temperatur kann zwischen einer Raumtemperatur von ca. 20°C bis zu einer erhöhten Temperatur von 42°C liegen. Als Eintauchzeiten können Zeitspannen von beispielsweise 5 bis 240 Sekunden gewählt werden.

Zusätzlich zu bzw. noch vor dem regulären alkalischen Beizen wird also ein zusätzliches alkalisches Beizen in einem Beizbad mit reduzierter physikalisch-chemischer Wirkung in das Verfahren integriert, so dass beim Eintauchen von weichfleckigkeitsbehafteten Bauteilen nur die "weichen Flecken" ohne eine Ausbildung der kompakten schwarzen Schicht erkennbar werden. Nach dem Eintauchen des Bauteils in das zusätzliche Beizbad braucht es lediglich gespült zu werden, um im Anschluss daran direkt einer Sichtprüfung unterzogen zu werden. Auch dieses Verfahren läuft also "nass in nass" ab.

Wenn die Sichtprüfung einen positiven Befund, also die Entdeckung einer Weichfleckigkeit lieferte, kann zunächst lediglich eine Bauteilabweichung festgestellt werden, ohne das Bauteil sofort dem Ausschuss zuzuordnen. An die Feststellung einer Bauteilabweichung kann sich ein standardisierter technischer Überprüfungsprozess anschließen, in dem nach definierten Kriterien untersucht und entschieden wird, ob das Bauteil dennoch verwendet werden kann ("use as is"), ob es repariert werden kann oder ob es dem Ausschuss zugeordnet wird. Dauert dieser Prozess länger, beispielsweise einen oder mehrere Tage, und kann das Bauteil, das weiterverwendet oder repariert werden kann, während dieser Zeit nicht "nass" gehalten werden, dann kann es den vollständigen, normalen Vorprozess noch einmal durchlaufen, diesmal aber ohne Unterbrechung. Damit kann sichergestellt werden, dass auch ein Bauteil, das in der Sichtprüfung einen Befund ergab, dennoch aber weiterverwendet werden kann, den gleichen Qualitätskriterien genügt, wie ein Bauteil ohne Befund.

Das alkalische Reinigen, das alkalische Beizen und das saure Beizen sowie die anschließenden Schritte des Anodisierens lassen sich weitgehend mechanisiert und ggf. automatisiert durchführen. Nach einer weiteren vorteilhaften Ausgestaltung der Erfindung kann auch die Sichtprüfung auf Weichfleckigkeit automatisiert durchgeführt werden. Dazu können die zu prüfenden Bauteile von einer Kamera optisch erfasst und die aufgenommenen Bilder von einer Bilderkennungssoftware auf Farbunterschiede hin untersucht und ausgewertet werden. Dadurch kann die Sichtprüfung objektiviert und nachprüfbar protokolliert werden, womit sie sich problemlos nicht nur in den Ablauf des Anodisierungsverfahrens, sondern auch in einen Prozess der Qualitätssicherung integrieren lässt. Außerdem kann dadurch der Einsatz eines Bearbeiters zur Sichtprüfung im Umfeld z. T. aggressiver Säuren und Laugen eingespart werden.

Nach einer weiteren vorteilhaften Ausgestaltung des erfinderischen Verfahrens kann nach dem letzten Spülen des Vorprozesses eine Risseindringprüfung und/oder ein Oberflächenschutzverfahren nach einem der oben genannten Prinzipien durchgeführt werden. Da das Bauteil bei der Risseindringprüfung mit einem Eindringmittel benetzt wird, das einem Oberflächenschutzverfahren hinderlich im Wege steht, kann es nach erfolgreichem Abschluss der Risseindringprüfung erneut einem vollständigen Vorprozess unterworfen werden. Ohne eine derartige Risseindringprüfung kann es unmittelbar einem Oberflächenschutzverfahren zugeführt werden ohne das Risiko, aufgrund von Weichfleckigkeit unnötig behandelt und später ausgesondert zu werden.

Die eingangs genannte Aufgabe wird außerdem bei dem Verfahren zur Prüfung von Bauteilen aus Aluminium oder Aluminiumlegierungen auf Weichfleckigkeiten in einem Oberflächenbehandlungsverfahren, das einen Vorprozess mit einem alkalischen Beizen und einem sauren Beizen als Teilprozess enthält, dadurch gelöst, dass vor dem alkalischen Beizen und/oder vor dem sauren Beizen eine Sichtprüfung auf Weichfleckigkeit vorgenommen wird. Dieses Verfahren zeigt die oben bereits erläuterten Vorteile und lässt sich in der oben geschilderten Weise variieren.

Im Folgenden wird das Prinzip der Erfindung anhand einer Zeichnung beispielshalber noch näher erläutert. In der Zeichnung zeigen:
- Figur 1:: einen Vorprozess nach dem Stand der Technik,
- Figur 2:: das Verfahren in seiner allgemeinsten Form,
- Figur 3:: eine nicht erfindungsgemäße Variante des Verfahrens für spezielle Aluminiumlegierungen,
- Figur 4:: eine nicht erfindungsgemäße Variante des Verfahrens mit einer Sichtprüfung vor dem alkalischen Beizen,
- Figur 5:: das erfindungsgemäße Verfahren mit einer Sichtprüfung vor dem sauren Beizen,
- Figur 6:: das erfindungsgemäße Verfahren mit einem vorherigen Trocknungsschritt,
- Figur 7:: eine konkrete Ausführungsform des Verfahrens nach Figur 6.

Ein herkömmlicher Vorprozess für ein an sich bekanntes Oberflächenschutzverfahren für Bauteile aus Aluminium oder Aluminiumlegierungen beginnt mit Entfettungs- und Beizbädern, die basische und saure Beizschritte umfassen und die einer Reinigung des Bauteils dienen. Zwischen den Reinigungs- und Beizbädern erfolgt ein Zwischenspülen, um die Chemikalien der Bäder nicht in das nachfolgende Bad zu verschleppen. Das Bauteil bzw. mehrere Bauteile werden dazu an einem Gestell aufgehängt, um in Tauchbädern behandelt zu werden. Damit lassen sich die Bearbeitungsschritte vorteilhaft automatisieren, um Bediener möglichst wenig mit den Chemikalien der Tauchbäder in Kontakt zu bringen. Für eine verbesserten Wirkungsgrad der Reinigungs- und Beizbäder können die Bauteile oder die Bäder bewegt werden, letzteres beispielsweise durch Rühren, Einblasen von Druckluft oder Rütteln des Behälters.

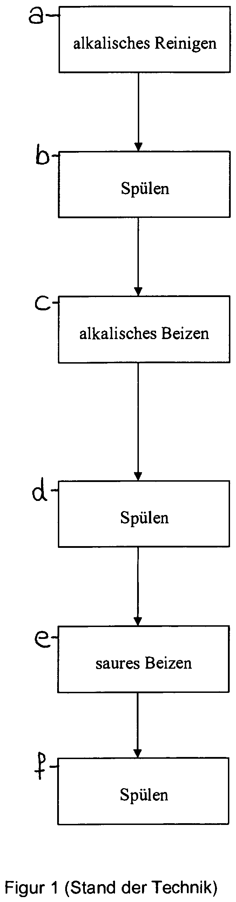

Auf ein "alkalisches Reinigen" der Bauteile in einem ersten Schritt a) erfolgt gemäß Figur 1 ein "Spülen" in einem Schritt b), um keine Flüssigkeitsreste aus dem Reinigungsschritt a) in die nachfolgenden Schritte zu verschleppen. Anschließend erfolgt ein "alkalisches Beizen" in einem Schritt c). Dabei entsteht eine graubraune bis überwiegend schwarze Schicht auf dem behandelten Bauteil. In einem weiteren Schritt d) werden die Bauteile gespült und von den Resten der alkalischen Beize befreit. In einem anschließenden Schritt e) werden sie einer "sauren Beize" unterzogen. In einem daran anschließenden Schritt f) werden die Bauteile in bekannter Weise gespült, um anschließend einer Risseindringprüfung oder dem Oberflächenschutzverfahren unterworfen zu werden.

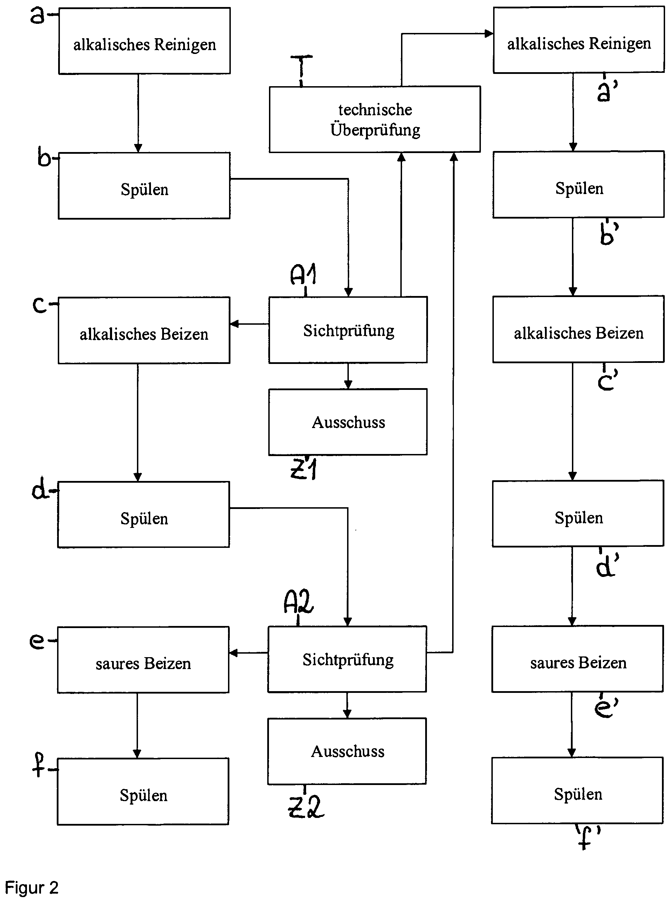

Figur 2 zeigt das Prinzip des Verfahrens in einer verhältnismäßig allgemeinen Darstellungsform. Sie verdeutlicht, dass das Verfahren sich auf einen Vorprozess mit den Schritten a) bis f) gemäß Stand der Technik stützt, der weitgehend unverändert auf der linken Seite bzw. als linke Spalte dargestellt ist. Das Prinzip der Erfindung beruht darauf, dass der Vorprozess nach dem "alkalischen Reinigen" in Schritt a) bzw. nach dem ihm folgenden "Spülen" in Schritt b) und/oder nach dem "alkalischen Beizen" in Schritt c) bzw. dessen Nachfolgeschritt d), dem "Spülen", unterbrochen wird, um eine Sichtprüfung A1 und/oder A2 durchzuführen. Prinzipiell werden also in allen Verfahrensvarianten der Erfindung die Verfahrensschritte a) bis f) an sich unverändert durchgeführt, wobei zwischen den Schritten b) und c) und/oder zwischen den Schritten d) und e) zusätzliche Schritte im Zusammenhang mit einer Sichtprüfung A bzw. A1 und/oder A2 eingeschoben werden.

Die Erfindung beruht in einem ersten Aspekt auf der Erkenntnis, dass sich an Stellen, an denen während eines vorangegangenen mechanischen Bearbeitungsschritts des Bauteils eine unzulässige lokale Überhitzung stattgefunden hat, sich milchig-weiß bis leicht bräunliche Flecken bilden. Anhand der braunen Flecken können die überhitzten Stellen, die zu einer lokal begrenzten geringeren Härte des Bauteils, den so genannten "weichen Flecken" führen, optisch identifiziert werden. Die "weichen Flecken" werden schon nach dem "alkalischen Reinigen" in Schritt a) bzw. nach dem anschließenden "Spülen" in Schritt b) sichtbar. Der Vorprozess lässt sich also bereits nach dem Schritt a) oder nach dem Schritt b) unterbrechen, um das Bauteil auf Weichfleckigkeit zu untersuchen. Je früher ein geschädigtes Bauteil identifiziert und aus seinem Verarbeitungsprozess ausgesondert werden kann, umso weniger unnötige Kosten verursacht es. Der frühe Zeitpunkt der Erfassung einer eventuellen Weichfleckigkeit trägt also zu einer Kostenreduktion des Verarbeitungsprozesses bei.

Sofern die Sichtprüfung A1 gemäß Figur 2 einen zweifelsfreien positiven Befund, also eine nicht akzeptable Weichfleckigkeit des untersuchten Bauteils zeigt, wird es dem Ausschuss Z1 zugeführt. In Zweifelsfällen jedoch kann das Bauteil nach der Sichtprüfung A1 einem standardisierten technischen Überprüfungsprozess T unterzogen werden, in dem untersucht wird, ob die festgestellte Bauabweichung im konkreten Fall toleriert werden kann (auch als "use as is" bezeichnet), ob sie reparabel ist oder ob sie tatsächlich irreparabel und damit schrottreif ist. Der technische Überprüfungsprozess kann länger dauern, beispielsweise einen oder mehrere Tage, so dass das Bauteil während dieser Zeit nicht nass gehalten werden kann. Er bewirkt, dass das Bauteil nicht unverändert dem laufenden Vorprozess, also dem an sich anschließenden Schritt c) "alkalisches Beizen" zugeführt werden kann. Das Bauteil muss vielmehr erneut dem Schritt a') "alkalisches Reinigen" dem Schritt b') "Spülen" unterzogen werden. Der Übersichtlichkeit halber ist ein vollständiger Vorprozess auf der rechten Seite bzw. in der rechten Spalte der Figur 2 dargestellt, der sich aber aus denselben Schritten a) bis f) gemäß der linken Spalte der Figur 2 zusammensetzt. Faktisch wird also das "alkalische Reinigen" des Schritt a) als Schritt a') wiederholt und nach Schritt b) bzw. b') gespült, wonach der unterbrochene Vorprozess der linken Spalte in Schritt c') mit dem "alkalische Beizen" fortgesetzt wird.

Sollte sich in der Sichtprüfung A1 kein positiver Befund zeigen, wird der Vorprozess mit dem bekannten Schritt c) "alkalisches Beizen" fortgesetzt. Denn ein positiver Befund in der Sichtprüfung A1 zeigt sich umso eher, je ausgeprägter der schädigende Wärmeeintrag in das Bauteil war. War die Überhitzung weniger stark, zeigt sie sich eventuell erst in einem späteren Verfahrensschritt. Sicherheitshalber wird daher nach dem anschließenden Schritt d) "Spülen" eine weitere Sichtprüfung A2 vorgenommen.

Denn beim "alkalischen Beizen" in Schritt c) entsteht auf dem behandelten Bauteil eine graubraune bis überwiegend schwarze Schicht (im Folgenden der Einfachheit halber als "schwarze Schicht" bezeichnet). Der Erfindung ist es zu verdanken, als weiteren Aspekt erforscht zu haben, dass an "weichen Flecken" des Bauteils sich in der schwarzen Schicht leicht bräunliche Flecken bilden. Anhand der braunen Flecken können die überhitzten Stellen, die zu einer lokal begrenzten geringeren Härte des Bauteils führen, optisch identifiziert werden. Die saure Beize in Schritt e) würde diesen Oberflächeneffekt aber wieder zunichte machen. Das Bauteil wird ausschließlich nach dem "alkalischen Beizen" in Schritt c) oder dem anschließenden "Spülen" in Schritt d) einer Sichtprüfung A2 unterzogen, um es auf Weichfleckigkeit zu untersuchen. Nach der Sichtprüfung in A2 wie nach derjenigen in Schritt A1 kann es entweder sofort dem Ausschuss Z2 zugeordnet werden oder der technischen Überprüfung T auf Bauteilabweichung unterworfen werden. Seine weitere Behandlung nach der technischen Überprüfung T entspricht insoweit derjenigen wie oben beschrieben, als wiederum das "alkalische Reinigen" als Schritt a') und das "Spülen" als Schritt b') wiederholt werden, jetzt aber zusätzlich auch das "alkalische Beizen" als Schritt c'), das "Spülen" als Schritt d') und das "saure Beizen" als Schritt e') angeschlossen werden, um den Vorprozess mit dem "Spülen" des Schritt f) bzw. f) zu beenden.

Sollte das Bauteil auch in der Sichtprüfung A2 keinen Befund liefern, erfährt es nach dem "sauren Beizen" in Schritt e) und dem anschließenden "Spülen" in Schritt f) den Abschluss des Vorprozesses.

Anschließend wird es einer Risseindringprüfung und/oder dem geplanten Oberflächenschutzverfahren zugeführt.

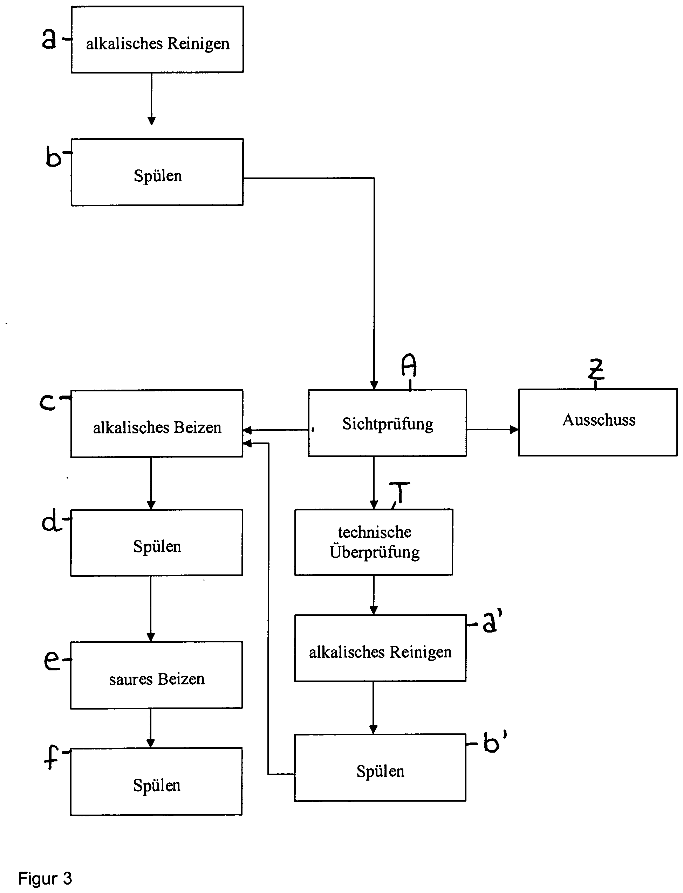

Figur 3 beschreibt ein vereinfachtes Verfahren nicht erfindungsgemäß für die Aluminiumlegierung AA2196.

Erfindungsgemäß konnte festgestellt werden, dass sich bei dieser Legierung bereits nach dem "alkalischen Reinigen" in Schritt a) und dem zugehörigen "Spülen" in Schritt b) alle "weichen Flecken" zeigen. Demnach ist die Sichtbarkeit der "weichen Flecken" vom Ausmaß der vorher eingebrachten Überhitzung in das Bauteil, von der Wärmeempfindlichkeit des Werkstoffes, also seiner Temperaturstabilität und von den Legierungsbestandteilen abhängig. Bereits die Sichtprüfung A nach dem "Spülen" in Schritt b) zeigt also zuverlässig einen Befund bei einem geschädigten Werkstück. Damit kann es entweder dem Ausschuss Z oder einer technischen Überprüfung T und damit dem erneuten "alkalischen Reinigen" in Schritt a') und dem zugehörigen "Spülen" in Schritt b') oder ohne Befund unmittelbar dem "alkalischen Beizen" in Schritt c) und dem weiteren Vorprozess mit den Schritten d) bis f) zugeführt werden. Für diese Legierung jedenfalls ergibt sich damit ein vereinfachtes und damit noch kostengünstigeres Prüfverfahren auf Weichfleckigkeit.

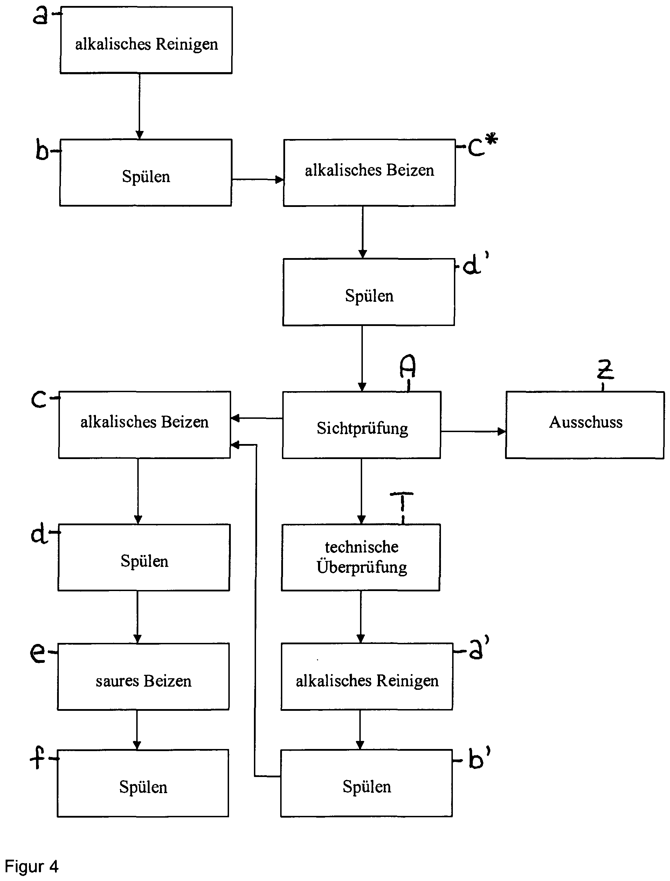

Wie bereits erwähnt führt das alkalische Beizen der Bauteile zu einer graubraunen bis überwiegend schwarzen Schicht, von der sich die "weichen Flecken" durch etwas hellere, leicht bräunliche Flecken abheben können. Je nach verwendeten Beizmitteln, ihrer Konzentration, der Temperatur des Beizbades, der Einwirkzeit und der Legierung des Bauteils können die "weichen Flecken" deutlicher oder weniger deutlich zu erkennen sein. Figur 4 zeigt daher eine nicht erfindungsgemäße Variante des Verfahrens, das nach dem "alkalischen Reinigen" in a) und dem zugehörigen "Spülen" in Schritt b) und vor der Sichtprüfung A einen weiteren Schritt c*) für ein "alkalisches Beizen" und einen dazugehörigen Schritt d') für ein "Spülen" vorsieht. Erfindungsgemäß wird für das "alkalische Beizen" des Schritts c*) grundsätzlich das gleiche Beizmittel wie für das "alkalische Beizen" in Schritt c) des Vorprozesses verwendet, jedoch mit einer verringerten Konzentration bei weitgehend gleicher Einwirkzeit und gleicher Temperatur. Entscheidend ist, dass das "alkalische Beizen" in Schritt c*) eine geringere physikalisch-chemische Wirkung auf das Aluminiumbauteil ausübt und damit das Sichtbarmachen der "weichen Flecken" bzw. der sie anzeigenden bräunlichen Flecken ohne Entstehung der schwarzen Schicht bewirkt. Dadurch kann in der anschließenden Sichtprüfung A ein zuverlässigeres Ergebnis erzielt werden, so dass das geprüfte Bauteil wie gehabt entweder ohne Befund dem unterbrochenen Vorprozess bei Schritt c) mit dem "alkalischen Reinigen" und den anschließenden Schritten d) bis f) zugeführt werden kann, oder bei einem Befund nach einer technischen Überprüfung T einem erneuten alkalischen Reinigen in Schritt a'), einem zugehörigen "Spülen" im Schritt b') und anschließend den Vorprozess mit dem Schritt c) fortsetzt oder im Ausschuss Z landet.

Geeignete Parameter für demgegenüber variierte Rezepturen mit unterschiedlichen Konzentrationen, Temperaturen und Einwirkzeiten der alkalischen Beize gemäß Schritt c*) sind der folgenden Tabelle zu entnehmen:

**Tabelle 1: beispielhafte Rezepturen für Schritt c*)**

| Konzentration | Temperatur | Einwirkzeit |
|---|---|---|
| 36 g/l | 42°C +/- 2°C | 20 sec. |
| 18 g/l | 20°C +/- 2°C | 90 sec. |
| 9 g/l | 20°C +/- 2°C | 180 sec. |

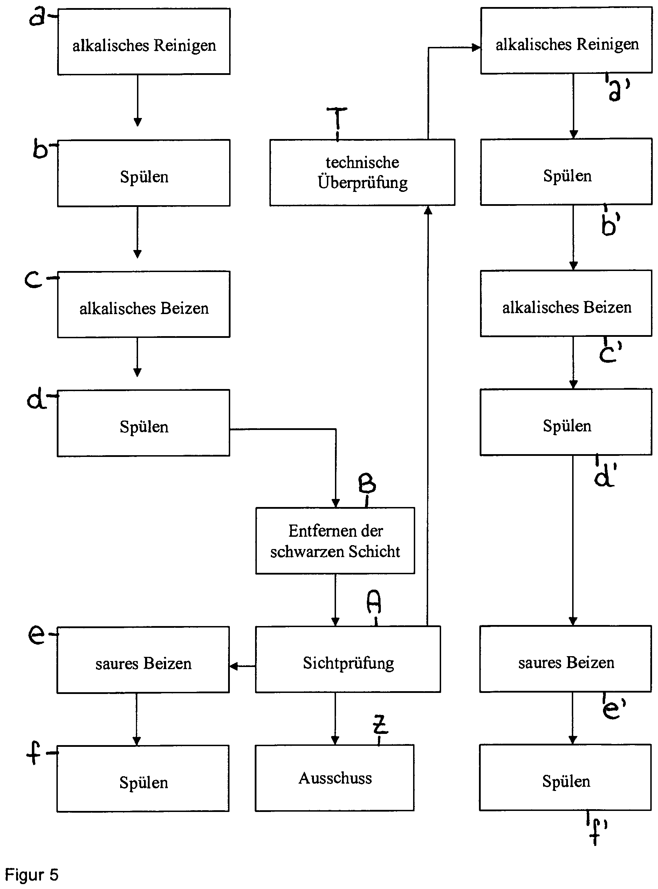

Figur 5 zeigt das erfindungsgemäße Verfahren, das die Unterbrechung des Vorprozesses nicht vor dem "alkalischen Beizen" in Schritt c) sondern vor dem "sauren Beizen" in Schritt e) vornimmt. Wie erwähnt führt das "alkalische Beizen" in Schritt c) zu der schwarzen Schicht auf dem behandelten Bauteil, in der sich die geschädigten überhitzten Stellen als leicht bräunliche Flecken zeigen können. Anders als das vorherige Verfahren sorgt das erfindungsgemäße Verfahren nach Figur 5 dadurch für eine gesteigerte Deutlichkeit der braunen Flecken, dass es nach dem "Spülen" in Schritt d) und vor der Sichtprüfung A die schwarze Schicht in einem Schritt B zumindest teilweise entfernt. Die Besonderheit dieses Verfahrensschritts liegt darin, dass die Intensität der Bearbeitung nur soweit reicht, dass lediglich die schwarze Schicht entfernt wird, nicht jedoch die braunen Flecken. Dazu kann Pressluft bzw. Druckluft eingesetzt werden. Aber auch Wasserstrahlen, Sandstrahlen, mechanische Bearbeitungsverfahren wie Bürsten oder der Einsatz von Vibration bzw. Schall oder spezieller Reinigungsmittel zum Entfernen der schwarzen Schicht sind denkbar. Je nach Auswahl des konkreten Verfahrens zum Entfernen der schwarzen Schicht kann es zusammen mit dem vorangegangenen Verfahrensschritt d) zum Spülen der Bauteile zusammengefasst werden oder ihn erübrigen. So können Düsen im Spülbad für Schritt d) eine Vielzahl von Wasserstrahlen unter Druck auf das Bauteil lenken, die die schwarze Schicht zumindest teilweise abtragen.

Entscheidend ist jedenfalls, dass das angewendete Verfahren im Verfahrenschritt B lediglich die schwarze Schicht oder nur einen Teil von ihr soweit abträgt, dass ein deutlicher Farbunterschied zwischen ihr bzw. der von ihr befreiten Flächen des Bauteils und den braunen Flecken erkennbar ist. Dazu ist also eine Schicht von wenigen Mikrometern abzutragen, etwa im Bereich von 0,1 bis 10 µm.

Nach Entfernen zumindest eines Teils der schwarzen Schicht ist das Bauteil vorbereitet für eine Sichtprüfung in dem anschließenden Schritt A. Jetzt können die braunen Flecken und damit die "weichen Flecken" des Materials der Bauteile identifiziert und lokalisiert werden, die Bauteile gekennzeichnet und sofort oder später ausgesondert werden. Neben einer visuellen Überprüfung der Bauteile durch geschultes Fachpersonal kann sie auch mit Hilfe von Kameras erfolgen und damit automatisiert werden.

Nach Abschluss der Sichtprüfung werden jedenfalls die nicht ausgesonderten Bauteile in der bekannten Weise weiterverarbeitet, nämlich in einem anschließenden Schritt e) einer sauren Beize unterzogen. In einem daran anschließenden Schritt f) werden die Bauteile in bekannter Weise gespült, um anschließend einer Risseindringprüfung oder dem Oberflächenschutzverfahren unterworfen zu werden.

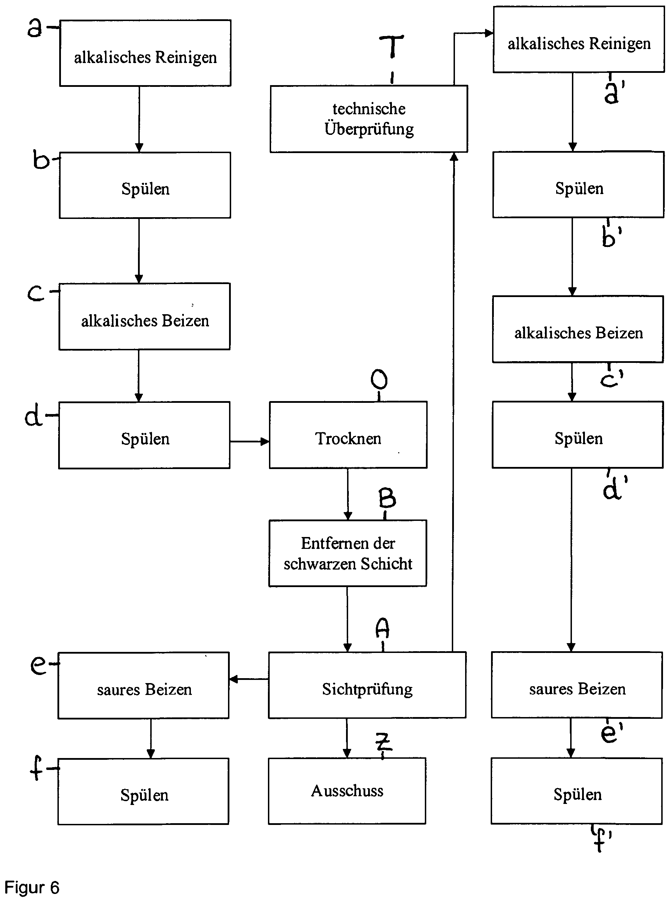

Figur 6 zeigt eine Alternative zum vorangehenden Verfahren gemäß Figur 5, das sich eine weitere Erkenntnis zur Verdeutlichung der bräunlichen Flecken gegenüber der schwarzen Schicht zunutze macht: bereits nach dem "alkalischen Beizen" in c) und dem anschließenden "Spülen" in Schritt d) sind bei manchen Aluminiumlegierungen ausreichende farbliche Unterschiede zu erkennen, wenn das Bauteil getrocknet wird. Erfindungsgemäß wird daher der Vorprozess nach dem Schritt d) unterbrochen: In einem Schritt O werden die Bauteile zunächst getrocknet. Er dient dazu, die Bauteile von der Feuchtigkeit aus dem Spülvorgang in Schritt d) zu befreien. Das Trocknen kann in bekannter Weise durch Lagern in Warmluft erfolgen und durch den Einsatz von öl- und wasserfreier Pressluft bzw. Druckluft unterstützt werden. Auch ein Spülen mit Warmwasser im vorangegangenen Schritt d) kann zu einem schnelleren Trocknungsergebnis führen. Je nach Auswahl des konkreten Verfahrens zum Entfernen der schwarzen Schicht in Schritt B kann es zusammen mit dem vorangegangenen Verfahrensschritt O zum Trocknen der Bauteile zusammengefasst werden oder ihn erübrigen. Wird beispielsweise Pressluft zum Entfernen der schwarzen Schicht eingesetzt, führt sie zugleich zum Trocknen der Bauteile, so dass die Schritte O und B zusammenfallen.

An die Sichtprüfung A schließen sich die bekannten Verfahrensschritte an, wonach ein Bauteil ohne Befund dem Vorprozess und dort dem "sauren Beizen" in Schritt e) und dem anschließenden "Spülen" im Schritt f) zugeführt wird. Ein Bauteil mit eindeutigem Befund kann dem Ausschuss Z zugeordnet werden oder der technischen Überprüfung T auf Bauteilabweichung, die zur Wiederholung der ersten Schritte a) bis d) des Vorprozesses erfordert.

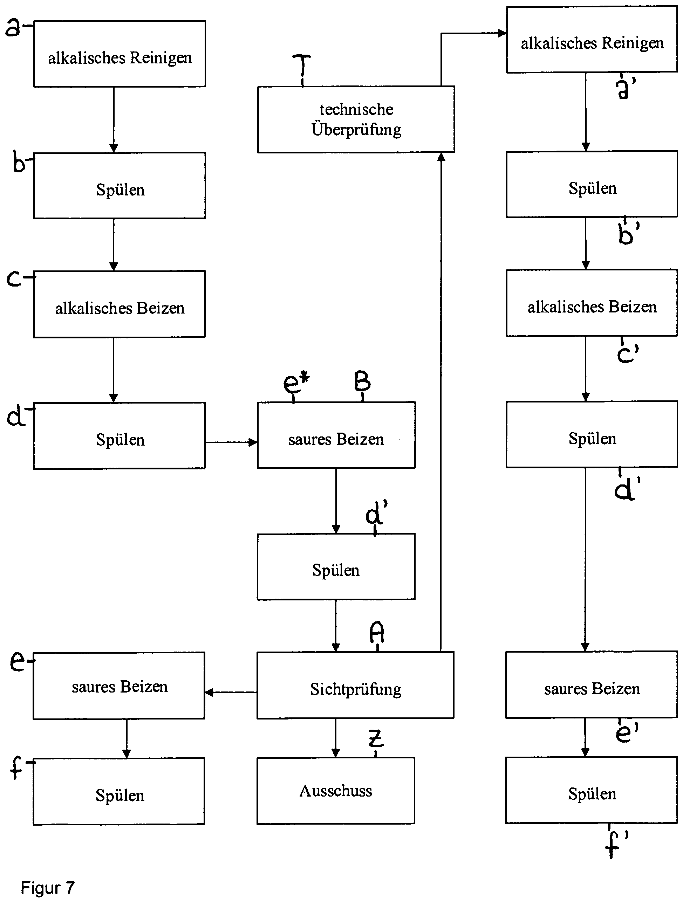

Figur 7 zeigt eine weitere Variante des Verfahrens gemäß Figur 5. Sie macht sich die Erkenntnis zunutze, dass das "saure Beizen" in Schritt e) die schwarze Schicht beseitigt. Erfindungsgemäß wird daher nach dem "Spülen" in d) das Verfahren vor der Sichtprüfung A mit einem Schritt e*) unterbrochen, in dem ein "saures Beizen" mit verringerter Konzentration stattfindet. Dieser Verfahrensschritt nutzt die gleiche Beize wie im Vorprozess des Schritts e), führt aber durch die verringerte Konzentration zu einem nur teilweisen Abtrag der schwarzen Schicht, lässt aber die bräunlichen Flecken weitgehend unberührt. Der Schritt e*) ersetzt folglich den Schritt B gemäß Figur 4, in dem die schwarze Schicht zumindest teilweise entfernt wird. Nach einem anschließenden Schritt d'), in dem die Bauteile gespült werden, können sie der Sichtprüfung A zugeführt werden. Das weitere Verfahren entspricht demjenigen gemäß Figur 5.

Da es sich bei den vorhergehenden, detailliert beschriebenen Verfahren um Ausführungsbeispiele handelt, können sie in üblicher Weise vom Fachmann in einem weiten Umfang modifiziert werden, ohne den Bereich der Erfindung zu verlassen. Insbesondere können die angegebenen konkreten Rezepturen der Reinigungs- und Beizbäder in anderer Weise als in der hier beschriebenen zusammengesetzt sein und vor allem hinsichtlich der Konzentrationen, Temperaturen und Einwirkzeiten variiert werden. Ebenso können Vorgänge wie das Eintauchen der Bauteile in andere Form, beispielsweise durch Besprühen oder Wischen, oder das Spülen nicht nur einmal, sondern mehrmals erfolgen, wenn sich dies zum Beispiel aus verfahrenstechnischen Gründen anbietet. Schließlich schließt die Verwendung der unbestimmten Artikel "ein" bzw. "eine" nicht aus, dass die betreffenden Merkmale auch mehrfach bzw. mehrmals vorhanden sein können.

### Bezugszeichenliste

- a), a'): alkalisches Reinigen
- b), b'): Spülen
- c), c'): alkalisches Beizen
- d), d'): Spülen
- e), e'): saures Beizen
- f), f): Spülen

- c*): alkalisches Beizen mit verminderter Konzentration
- e*): saures Beizen mit verminderter Konzentration

- A, A1, A2: Sichtprüfung
- B: Entfernen der Oxidschicht
- O: Trocknen
- T: technische Überprüfung
- Z, Z1, Z2: Ausschuss

## Patentansprüche

1. Oberflächenbehandlungsverfahren für Bauteile aus Aluminium oder Aluminiumlegierungen umfassend einen Vorprozess mit den folgenden Behandlungsschritten:
a) alkalisches Reinigen,
b) Spülen,
c) alkalisches Beizen,
d) Spülen,
e) saures Beizen,
f) Spülen,
wobei nach dem Spülen im Schritt b) und/oder nach dem Spülen im Schritt d) ein Schritt (A; A1; A2) einer visuellen Sichtprüfung auf Weichfleckigkeit durchgeführt wird, **dadurch gekennzeichnet, dass** nach dem Spülen im Schritt d) und vor dem Schritt (A; A2) der visuellen Sichtprüfung in einem separaten Schritt (B) eine durch das alkalische Beizen entstandene schwarze Schicht auf den Bauteilen wenigstens teilweise entfernt wird.

2. Verfahren nach Anspruch 1, **dadurch gekennzeichnet, dass** vor dem Schritt (A;
A2) der Sichtprüfung ein zusätzlicher Schritt (O) zur Trocknung des Bauteils vorgenommen wird.

3. Verfahren nach Anspruch 2, **dadurch gekennzeichnet, dass** die Trocknung im zusätzlichen Schritt (O) mittels Druckluft erfolgt.

4. Verfahren nach Anspruch 1, **dadurch gekennzeichnet, dass** das Entfernen der schwarzen Schicht in Schritt B durch mechanische Verfahren oder durch chemische Mittel erfolgt.

5. Verfahren nach Anspruch 1 oder Anspruch 4, soweit dieser sich auf chemische Mittel bezieht, **dadurch gekennzeichnet, dass** das Entfernen der schwarzen Schicht mit einer sauren Beize mit verringerter Konzentration und/oder verringerter Einwirkzeit und/oder verringerter Temperatur erfolgt.

6. Verfahren nach einem der Ansprüche 1 bis 3, **dadurch gekennzeichnet, dass** nach dem alkalischen Reinigen in Schritt a) und vor der Sichtprüfung in Schritt (A) in einem zusätzlichen Schritt c*) ein alkalisches Beizen mit einer Beize mit geringerer Konzentration und/oder verringerter Einwirkzeit und/oder verringerter Temperatur verwendet wird.

7. Verfahren nach einem der obigen Ansprüche, **dadurch gekennzeichnet, dass** zwischen der Sichtprüfung (A1) und dem alkalisches Beizen in Schritt c) die Schritte a') alkalisches Reinigen und b') Spülen durchgeführt werden.

8. Verfahren nach einem der obigen Ansprüche, **dadurch gekennzeichnet, dass** zwischen der Sichtprüfung (A2) und dem sauren Beizen in Schritt d) die Schritte a') alkalisches Reinigen, b') Spülen, c') alkalisches Beizen und d') Spülen durchgeführt werden.

9. Verfahren nach einem der obigen Ansprüche, **dadurch gekennzeichnet, dass** sich nach der Sichtprüfung (A; A1; A2) bei Bauteilen mit einem positiven Befund ein standardisierter technischer Überprüfungsprozess anschließt.

10. Verfahren nach einem der obigen Ansprüche, **dadurch gekennzeichnet, dass** die Sichtprüfung (A; A1; A2) automatisiert durchgeführt wird.

11. Verfahren nach einem der obigen Ansprüche, **dadurch gekennzeichnet, dass** nach dem Schritt f) eine Risseindringprüfung und/oder ein Oberflächenschutzverfahren durchgeführt wird.

12. Verfahren zur Prüfung von Bauteilen aus Aluminium oder Aluminiumlegierungen auf Weichfleckigkeit in einem Oberflächenbehandlungsverfahren nach einem der obigen Ansprüche, **dadurch gekennzeichnet, dass** vor einem Schritt c) zum alkalischen Beizen und/oder vor einem Schritt e) zum sauren Beizen eine Sichtprüfung (A; A1; A2) auf Weichfleckigkeit vorgenommen wird.

## Claims

1. Surface treatment method for components made of aluminium or aluminium alloys, comprising a preliminary process with the following treatment steps:
a) alkaline cleaning,
b) rinsing,
c) alkaline pickling,
d) rinsing,
e) acidic pickling,
f) rinsing,
where after the rinsing in step b) and/or after the rinsing in step d), a step (A; A1; A2) of visual inspection for soft spots is carried out, **characterized in that** after the rinsing in step d) and before the step (A; A2) of visual inspection, in a separate step (B), a black layer on the components, resulting from the alkaline pickling, is at least partly removed.

2. Method according to Claim 1, **characterized in that** before the step (A; A2) of inspection, an additional step (0) is performed for drying the component.

3. Method according to Claim 2, **characterized in that** the drying in the additional step (0) takes place by means of compressed air.

4. Method according to Claim 1, **characterized in that** the removal of the black layer in step B takes place by mechanical methods or by chemical agents.

5. Method according to Claim 1 or Claim 4, in so far as the latter refers to chemical agents, **characterized in that** the removal of the black layer takes place with an acidic pickle having reduced concentration and/or reduced exposure time and/or reduced temperature.

6. Method according to any of Claims 1 to 3, **characterized in that** after the alkaline cleaning in step a) and before the inspection in step (A), in an additional step c*), an alkaline pickling is used with a pickle having lower concentration and/or reduced exposure time and/or reduced temperature.

7. Method according to any of the above claims, **characterized in that** between the inspection (A1) and the alkaline pickling in step c), the steps of a') alkaline cleaning and b') rinsing are carried out.

8. Method according to any of the above claims, **characterized in that** between the inspection (A2) and the acidic pickling in step d), the steps of a') alkaline cleaning, b') rinsing, c') alkaline pickling and d') rinsing are carried out.

9. Method according to any of the above claims, **characterized in that** the inspection (A; A1; A2) in the case of components with a positive finding is followed by a standardized technical examination process.

10. Method according to any of the above claims, **characterized in that** the inspection (A; A1; A2) is carried out automatedly.

11. Method according to any of the above claims, **characterized in that** after the step f), a crack penetration test and/or a surface protection method are/is carried out.

12. Method for examining components made of aluminium or aluminium alloys for soft spots in a surface treatment method according to any of the above claims, **characterized in that** before a step c) for alkaline pickling and/or a step e) for acidic pickling, an inspection (A; A1; A2) for soft spots is performed.

## Revendications

1. Procédé de traitement de surface pour composants en aluminium ou alliages d'aluminium, comprenant un procédé préliminaire qui comprend les étapes de traitement suivantes :
a) un nettoyage alcalin,
b) un rinçage,
c) un décapage alcalin,
d) un rinçage,
e) un décapage acide,
f) un rinçage,
une étape (A ; A1 ; A2) d'inspection visuelle des points faibles étant réalisée après le rinçage à l'étape b) et/ou après le rinçage à l'étape d), **caractérisé en ce qu'**après le rinçage à l'étape d) et avant l'étape (A ; A2) de l'inspection visuelle, lors d'une étape (B) séparée, une couche noire formée par le décapage alcalin sur les composants est au moins partiellement éliminée.

2. Procédé selon la revendication 1, **caractérisé en ce qu'**avant l'étape (A ; A2) de l'inspection visuelle, une étape (0) supplémentaire est réalisée pour le séchage du composant.

3. Procédé selon la revendication 2, **caractérisé en ce que** le séchage lors de l'étape supplémentaire (0) a lieu au moyen d'air comprimé.

4. Procédé selon la revendication 1, **caractérisé en ce que** l'élimination de la couche noire à l'étape B a lieu par des procédés mécaniques ou par des moyens chimiques.

5. Procédé selon la revendication 1 ou la revendication 4, dans la mesure où celle-ci concerne des moyens chimiques, **caractérisé en ce que** l'élimination de la couche noire a lieu avec un décapant acide à concentration réduite et/ou à temps d'action réduit et/ou à température réduite.

6. Procédé selon l'une quelconque des revendications 1 à 3, **caractérisé en ce qu'**après le nettoyage alcalin à l'étape a) et avant l'inspection visuelle à l'étape (A), lors d'une étape c*) supplémentaire, un décapage alcalin avec un décapant à concentration réduite et/ou à temps d'action réduit et/ou à température réduite est utilisé.

7. Procédé selon l'une quelconque des revendications précédentes, **caractérisé en ce qu'**entre l'inspection visuelle (A1) et le décapage alcalin à l'étape c), les étapes a') de nettoyage alcalin et b') de rinçage sont réalisées.

8. Procédé selon l'une quelconque des revendications précédentes, **caractérisé en ce qu'**entre l'inspection visuelle (A2) et le décapage acide à l'étape d), les étapes a') de nettoyage alcalin, b') de rinçage, c') de décapage alcalin et d') de rinçage sont réalisées.

9. Procédé selon l'une quelconque des revendications précédentes, **caractérisé en ce que** l'inspection visuelle (A ; A1 ; A2) est suivie par un procédé d'inspection technique normalisé pour les composants présentant un résultat positif.

10. Procédé selon l'une quelconque des revendications précédentes, **caractérisé en ce que** l'inspection visuelle (A ; A1 ; A2) est réalisée automatiquement.

11. Procédé selon l'une quelconque des revendications précédentes, **caractérisé en ce qu'**une inspection par pénétration et/ou un procédé de protection de surface sont réalisés après l'étape f).

12. Procédé d'inspection de composants en aluminium ou alliages d'aluminium en termes de points faibles dans un procédé de traitement de surface selon l'une quelconque des revendications précédentes, **caractérisé en ce qu'**avant une étape c) de décapage alcalin et/ou avant une étape e) de décapage acide, une inspection visuelle (A ; A1 ; A2) des points faibles est réalisée.
